# EUROPEAN PATENT APPLICATION

(11) **EP 1 864 629 A2**
(43) Date of publication of application: **12.12.2007**
(21) Application number: 07109649.9
(22) Date of filing: 05.06.2007
(51) Int. Cl.: A61F 2/38

(54) **Flexible joint implant**

(30) Priority: 07.06.2006 US 448954
(71) Applicant: Howmedica Osteonics Corp., Mahwah, NJ 07430 (US)
(72) Inventor: Rickels, Tracy, Florida 10921 (US); Krijger, Peter, Rockaway, NJ 07866 (US); Jones, Eric, Limerick (IE)
(74) Representative: Collin, Jérôme

(57) **Abstract**

A trochlear groove implant includes a body having an articulate-bearing surface, a bone-facing surface and an edge extending therebetween for repairing a trochlear groove. The articulate-bearing surface configured to mimic a portion of a normal trochlear groove. The implant further includes at least one anchoring mechanism extending outwardly from the body.

## Description

### BACKGROUND OF THE INVENTION

The present invention relates generally to a replacement device for a joint. One such joint where the present invention is particularly adapted for is the trochlear groove of a femur. Although the present invention may be used in conjunction with various joints, it will be described in reference to a knee joint.

The human knee joint primarily includes three parts, anatomically referred to as the femur, the tibia, and the patella. The knee joint is then further subdivided into two joints: the patella-femoral joint (spaced between the knee cap and distal anterior surface of the femur) and the tibial-femoral (spaced between the femur and the tibia).

During normal and straightening of the leg, the patella slides over the femur within a groove that is located on the front distal surface of the femur. This groove is referred to as a trochlear groove. Several types of abnormalities can occur with the movement of the patella over the femur. For example, the patella may dislocate or slip out of place, a fracture, or develop a tracking problem. Normally, the patella tracks, or glides within the central region of a trochlear groove. A tracking problem occurs when the patella no longer remains centered within a groove as it slides over the femur. The resulting abnormal biomechanics can cause chronic pain in the joint, and if left untreated, it can lead to degenerative arthritis. The distal end of the femur, within which resides a trochlear groove, is coated with articulate cartilage. This cartilage functions as a cushion between the femur and the tibia. In arthritis of the knee joint, the articulate cartilage breaks down either from abnormal wear as mentioned above, or from injury, age, congenital predisposition, inflammatory arthritis, or obesity. When this cartilage breaks down, the cushion is lost, resulting in pain swelling, bone spur formation, and/or decreased range of motion of the knee joint.

Due to the inability of damaged cartilage to repair itself after injury, the range of treatment for patients with unicompartmental disease involving the patella is limited. The most commonly prescribed treatments include soft tissue releases and/or realignment of the patella tendon, patellectomy, or the patella is completely removed, or a total knee replacement with a stabilized patello-femoral-tibial prosthesis. In certain instances, none of these procedures may be desirable or effective. For example, the soft tissue procedures may not work. A patient having undergone a patellectomy is left partially crippled due to the loss of the knee cap, which serves to hold the joint together. Additionally, these patients often still suffer from pain due to contact of the remaining tendon moving directly over the groove. A total knee replacement with a standardized prosthesis is also far from ideal because much of the femur must be carved away in order to "fit" the distal surface of the femur to the standardized prosthesis. Additionally, the patients are often young and are likely to require replacements of the prosthesis. Each revision operation is more difficult. Therefore, there is still a need for a better treatment of patients with degenerative arthritis of their patella femoral knee joint.

Similar joints have similar issues that have been discussed with the knee and thus further options are required so as to replace a portion of a joint without requiring a total revision of the joint.

### SUMMARY OF THE INVENTION

A trochlear groove implant including a body having an articulate-bearing surface, a bone-facing surface and an edge extending therebetween. The articulate-bearing surface configured to mimic a portion of a normal trochlear groove. The implant further includes at least one anchoring mechanism extending outwardly from the body. The at least one anchoring mechanism being at least partially attached to the body. The at least one anchoring mechanism may be disposed within a bone to anchor the body to the bone.

The articulate-bearing surface may have a shape similar to a substantial portion of the trochlear groove, such that when the trochlear groove implant is implanted, it extends from a lateral side of the trochlear groove to a medial side of the trochlear groove of a patient. The body of the trochlear groove implant may substantially extend from an anterior cortex of the femur to an intercondular notch of the femur and/or the implant is positioned within a sulcus of a trochlear groove.

The body of the trochlear groove implant may be shaped to similar to a portion of an articulating surface of a lateral side or a medial side of the trochlear groove such that the body of the implant may be implanted adjacent to either a lateral side of a trochlear groove or a medial side of a trochlear groove and the articulate-bearing surface of the body extends along only one side of the trochlear groove.

In one aspect of the present invention, the at least one anchoring mechanism includes a first portion for engaging the body and a second portion that has a porosity sufficient to permit bone ingrowth. In another aspect, the at least one anchoring mechanism includes a first portion integrally formed to the body and a second portion removeably attached to the first portion. The second portion may be disposed within a recess created in a trochlear groove and the first portion may be removably engaged to the second portion such that if so required the first portion may be disengaged from the second portion and the body including the first portion of the anchoring mechanism can be removed.

In an alternate aspect of the present invention, a method for repairing the trochlear groove is disclosed. The method includes preparing a trochlear groove to receive an implant by creating an implant receiving area in the trochlear groove. And inserting an implant into the implant receiving area. The implant having an articulate-bearing surface that is positioned within the implant receiving area so as to mimic a normal trochlear groove articulating surface. The implant further includes an anchoring device for anchoring the implant in the implant receiving area. The implant receiving area may extend from a lateral side of a trochlear groove to a medial side of a trochlear groove and/or may extend substantially from an anterior cortex of a femur to an intercondular notch. Also the may only be positioned within a sulcus of a trochlear groove.

### BRIEF DESCRIPTION OF THE DRAWINGS

FIG. 1 is a top perspective view of one embodiment of the present invention;

FIG. 2 is a bottom perspective view of the embodiment shown in FIG. 1;

FIG. 3 is a side cross-sectional view of an anchoring mechanism;

FIG. 4 is a front view of a tool used in the method of forming embodiments of the present invention;

FIG. 5 is a front view of a joint having an implant receiving area;

FIG. 6 is a front view of a joint having an implant inserted therein;

FIG. 6A is a front view of a joint having an implant inserted therein;

FIG. 7 is a bottom perspective view of one embodiment of the present invention;

FIGS. 8 and 9A are top perspective views of one embodiment according to the present invention;

FIGS. 9B-9C are top perspective views of embodiments according to the present invention;

FIG. 9D is a top perspective view of one embodiment according to the present invention;

FIG. 9E is a bottom perspective view of the embodiment shown in FIG. 9D;

FIG. 9F is a top perspective view of one embodiment according to the present invention;

FIG. 10 is a top perspective view of one embodiment of the present invention;

FIG. 11 is a bottom perspective view of the embodiment shown in FIG. 10;

FIG. 12 is a front perspective view of an implant inserted into a joint;

FIG. 13 is a side perspective view of an embodiment according to the present invention;

FIG. 14 is an illustration of the implant of FIG. 13 inserted into a joint;

FIG. 15 is a cross-sectional view of the embodiment of FIG. 13;

FIG. 16 is a top perspective view of an embodiment according to the present invention;

FIGS. 16A-16E are perspective views of various templates which are used in conjunction with the implants of the present invention;

FIG. 17 is a top perspective view of a burr template used in conjunction with the present invention;

FIGS. 18-23 are side perspective views of tools used in conjunction with the present invention;

FIG. 24 illustrates an implant according to the present invention used within an acetabulum;

FIG. 24A illustrates a cross-sectional side view of the implant shown in FIG. 24;

FIG. 25 illustrates an implant according to the present invention implanted into an ankle joint;

FIG. 26 illustrates an implant according to the present invention implanted within a femoral head; and

FIG. 27 illustrates an implant according to the present invention implanted within a humeral head.

The present invention relates to an apparatus and a method for reconstructing a joint surface and particularly the bearing surface of a joint such that a relatively smooth interaction between opposing bone members may be achieved.

One aspect of the present invention is to provide a replacement device for a patient's joint such as a knee joint that replicates as closely as possible the original kinematics of the patient's joint. For instance, the replacement device should substantially replicate the patient's trochlear tracing pattern of the femur to maintain the original articulating movement of the knee. To do so, unhealthy articular cartilage is removed as well as portions of bone and replaced with a replacement device particularly suited for a patient's femur so as to maintain as closely as possible the original articulating movement of the patella about the trochlear groove. In an alternate embodiment, the implant may simply blanket the unhealthy articular cartilage. As such, the present invention is suitable to be used in conjunction with the "OATS" method as well as with a Microfracture process. In either case, once the particular method or process has been preformed, an implant of the present invention may be blanketed onto the processed area so as to provide a smooth articulate surface.

In one aspect of the present invention, the replacement device is specifically designed so that the underside of a patella articulates about the femur approximately 2 to 6 mm. away, thereby mimicking how a patella on a healthy articular cartilage would articulate. The above feature may be accomplished by providing a trochlear groove that is formed by a replacement device, which mimics the exterior surface of a natural trochlear groove on the femur. Moreover, as further discussed below, the underside of the replacement device is partially embedded into the femur or displaced adjacent to the femur. And may include anchoring elements that allow the replacement device to be held in place.

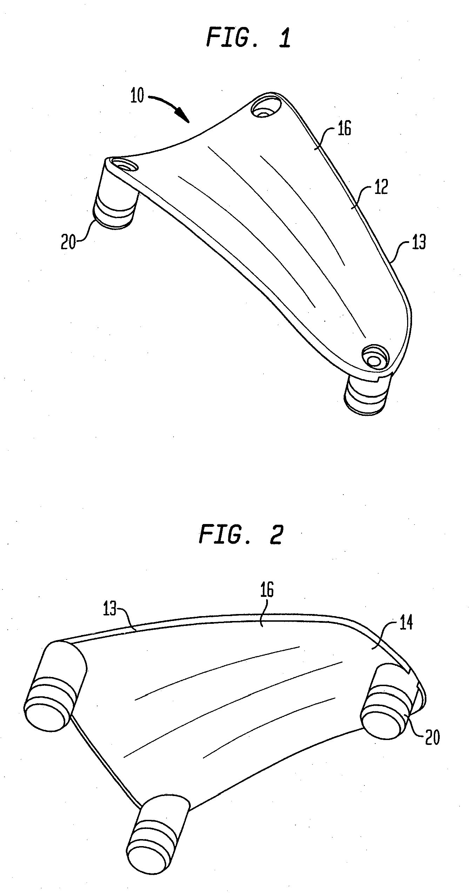

For example, a first embodiment of an orthopedic implant according to an aspect of the present invention is flexible joint implant 10 shown in FIGS. 1 and 2. The flexible joint implant 10 is particularly adapted to reconstruct a trochlear groove although the implant may be configured differently as required for other joints. Flexible joint implant 10 includes an articulate-bearing surface 12 and an oppositely-facing bone-contacting surface 14. The articulate-bearing surface 12 and the bone-contacting surface 14 comprise a body 16 of the flexible joint implant 10. An edge 13 extends from the articulate-bearing surface 12 to the bone-contacting surface 14. The body 16 is constructed from a low-profile layer of a smooth material such as a polymer including but not limited to, polyurethane and more particularly a polyurethane resin based upon a polycarbonate precursor. The material may also be a hydrogel such as polyvinylalcohol, which is especially adaptable to be used in a solution casting process or an injection molding process. And in one preferred embodiment, it may have a thickness of approximately between about 1 mm to 6 mm. The polymer articulate-bearing surface 12 enables the body 16 to mimic the feel and flexibility of normal cartilage found on the surface of joint structures.

Although the body 16 may be constructed from a single polymer, it may also comprise more than one polymer. For instance, the body 16 may be layered such that a soft polymer is disposed on top of a hard polymer or reverse. The composition may also include a 3 or even more layer composition such that the make up of the body goes from soft polymer, to hard polymer, to soft and back to hard as well as other combinations that may be required.

The flexible joint implant 10 also preferably includes an anchoring mechanism such as pegs 20 extending outwardly from bone-contacting surface 14. As shown in FIG. 1, in one preferred embodiment of the present invention 3 anchoring mechanisms, i.e., pegs 20, may be incorporated with the flexible joint implant 10 so as to provide a secure mount between the flexible joint implant and the bone to which it is anchored. In other embodiments, various numbers of anchoring mechanisms including one or more may be utilized to anchor the flexible joint implant 10.

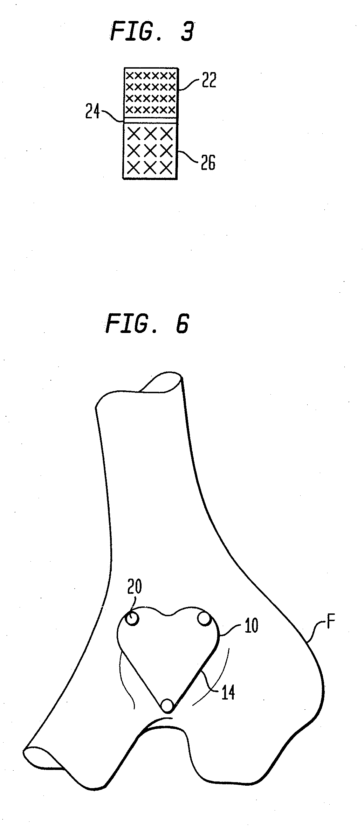

In one aspect of the present invention, the body 16 of the flexible implant 10 is partially embedded into the pegs 20 such that a stable connection may be maintained between the body 16 and the pegs 20. In order that this "embedding" may be employed, the pegs 20 are preferably constructed having a lattice-like structure, such as that shown in FIG. 3.

FIG. 3 illustrates an embodiment of a peg 20 having a polymer-engaging portion 22, an intermediate portion 24 and a bone-ingrowth portion 26. The polymer-engaging portion 22 is made up of various struts, walls, beams and appendages so as to form a matrix like lattice having a porosity The porosity of the polymer-engaging portion 22 is chosen so as best to aid in the incorporation of the polymer body 16 into and around the polymer-engaging portion 22. A preferred pore size may be approximately between 500 microns to 1200 microns, for a peg having a diameter of about 6 mm. Smaller or Larger diameter pegs may require a more proportionate pore size. As with the entire peg 20, the polymer-engaging portion 22 is preferably comprised of a biocompatible material such as but not limited to titanium or a similar metal structure.

The intermediate portion 24 of the peg 20, disposed between the polymer-engaging portion 22 and bone-ingrowth portion 26 preferably has a porosity that is significantly less than the porosity of the polymer-engaging portion 22 and low enough such that as the polymer body 16 is being attached to the polymer-engaging portion 22, as will be described below, the polymer material is unable to come in contact with the bone-ingrowth portion 26. The intermediate portion 24 acts as a barricade in preventing any leeching of the polymer material into the bone-ingrowth portion 26.

The bone-ingrowth portion 26 may be similarly constructed as the polymer-engaging portion 22 and be constructed with a particular porosity. Although the porosity of the bone-ingrowth portion 26 may vary, the preferred porosity is chosen so that when the peg is implanted in the bone, the bone-ingrowth portion 26 will promote bone ingrowth by the surrounding tissue. A preferred pore size for bone-ingrowth is approximately between 200 microns to 500 microns.

The peg 20 may be built using processes described in commonly assigned U.S. Patent Application Nos. 10/704,270 entitled, "Laser-Produced Porous Surface"; 11/027,421 entitled, "Gradient Porous Implant"; 11/295,008 entitled, "Laser-Produced Porous Surface"; and 60/755,260 entitled, "Laser-Produced Implants". As discussed in U.S. Patent Application No. 10/704,270, the metal structure, i.e., the peg, may be constructed using a selective laser melting or sintering process, which hereby grows the structure in a layer by layer process. In the alternative, the peg 20 may be built using an alternate process described in U.S. Patent Application No. 10/704,270 wherein the intermediate portion 24 acts as a base or substrate on which the polymer-engaging portion 22 and bone-ingrowth portion 26 are built thereon, also in a layer-by-layer fashion. Additional techniques for constructing the metal lattice, i.e., peg 20 may also be employed such as that disclosed in commonly assigned U.S. Patent Application No. 10/071,667 entitled, "Porous Metallic Scaffold for Tissue", as well as additional methods known to those in the art such as that disclosed in Patent Cooperation Treaty Application 2005/023118 entitled "Porous Metal Articles Formed Using an Extractable Particulate," filed on July 22, 2004.

The pegs 20 preferably have a height that is approximately between 2 to 15 mm. And the individual portions have heights that vary as for instance, the height of the polymer-engaging portion 22 is between 1 to 4 mm., thereby allowing the metal lattice to engage into the body 16 at a sufficient depth to anchor the body to the pegs 20. And the bone-engaging portion 26 preferably has a height approximately between 1 to 11 mm., such that the metal lattice of the bone engaging portion is sufficiently embedded into the bone to permit bone ingrowth to thereby anchor the pegs 20 within the surrounding bone. The intermediate portion 24 should be of sufficient height to prevent any of the polymer material used to construct the body 16 from leeching into the bone-engaging portion 26 as will be described below.

Although shown to have a cylindrical shape, the pegs 20 may have other geometric shapes such as polygonal and the like. And the pegs 20 may have protrusions or barb like extensions that anchor the pegs into a bone. The protrusions may be angled downwards so as to allow the pegs to easily enter a bore in a bone but limit their ability to be removed or slip out of the bore. The protrusion may also be angled sideways such that they limit rotation of the pegs 20 within a bore.

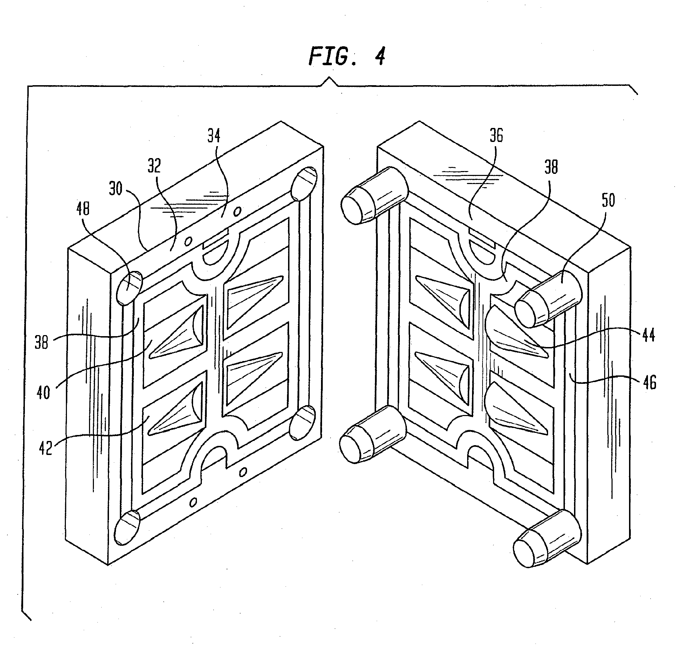

In a method of assembling the flexible joint implant 10 together an injection molding process may be utilized, which involves the use of a die 30 such as that shown in FIG. 4. In an alternate embodiment, not shown, a solvent casting method may be used, which is known to those in the art. The die 30 preferably includes a first component 32 having a front surface 34 and a second component 36 having a front surface 38. The front surfaces 34 and 38 respectively, are preferably engagable with one another, such that the two components 32 and 36 may be brought proximate to one another with the first surface 34 of first surface 32 confronting the first surface 38 of second component 36. In one aspect of the present invention, the first surface 34 of the first component 32 includes a plurality of molds 38. Each mold 38 may include a plurality of holes 40 disposed within a body-forming portion 42. The body-forming portion 42 is recessed within the molds 38 and preferably has a surface configuration substantially equivalent to the bone-contacting surface 14 of a flexible joint implant 10.

The first surface 36 of the second component 38 also includes a plurality of molds 44 and preferably includes an individual mold 44 for each individual mold 38 of the first component 32. The two molds of respective components may be thought of as having a male to female relationship, wherein when the first component 32 is brought proximate to the second component 36 such that the first surface 34 engages the first surface 38, an individual mold 38 of the first component 32 is engaged by a reciprocating individual mold 44 of second component 36 to form a single mold for an individual implant. Preferably, each mold 44 has a mold forming portion 46, that has a shape that is substantially equivalent to the surface shape of the articulate-bearing surface 12 of a flexible joint implant 10.

Employing the die 30 and its various features, flexible joint implants 10 may be created. To form the flexible joint implants 10, individual pegs 20 are each deposited within a hole 40 of the first component 32 disposed within a mold 38. Once the holes 40 are filled with pegs 20, the first component 32 may be engaged with the second component 36. Preferably, as shown in the figure, first component 32 has engagement elements such as recesses 48 and second component 34 has protrusions 50 which thereby permit a secure mating of the two die components. Once the die components have been assembled together, a melted polymer liquid material may be introduced into a passageway (not shown) disposed within the first surfaces 34 and 38 of the die components 32 and 36. The passageway allows the melted polymer, such as melted polyurethane to travel from outside the die components to in between the body-forming portions 42 and mold-forming portions 46 of the components 32 and 36.

According to one embodiment of the present invention, the melted polymer has a temperature of approximately 210°C. And the injection pressure of the polymer is less than 20 bar with a process time of 30 seconds. As the polymer melt is introduced into the die, the die may be sealed such that a pressure is applied to the polymer, thereby forcing the polymer melt into the holes 38 of the mold 40 and more specifically forcing the polymer to become embedded within the matrix of the polymer-engaging portion 22 of the pegs 20. Of course, as the polymer is forced into the polymer-engaging portion 22, the intermediate portion 24 prevents any of the polymer melt from leeching into the bone-ingrowth portion 26 thereby preventing the pores of the bone-ingrowth portion 26 from becoming blocked with the liquid. Once the optimum pressure between the two die components 32 and 36 has been achieved, the polymer is cooled and becomes solid, thereby forming the body 16 of the flexible joint implant 10.

Once the polymer has hardened, the first component 32 may be separated from the second component 36 using ejectors, not shown in the figures. And the now formed flexible joint implants may be removed from their respective molds. A cleaning process may be performed on each of the flexible joint implants, wherein any excess polymer around the edges of the implants may be removed by applying an abrasive treatment to the implant. Care should be taken not to damage the articulate-bearing surface 12 of each flexible joint implant 10 so as not to damage the smooth surface of the articulate-bearing surface.

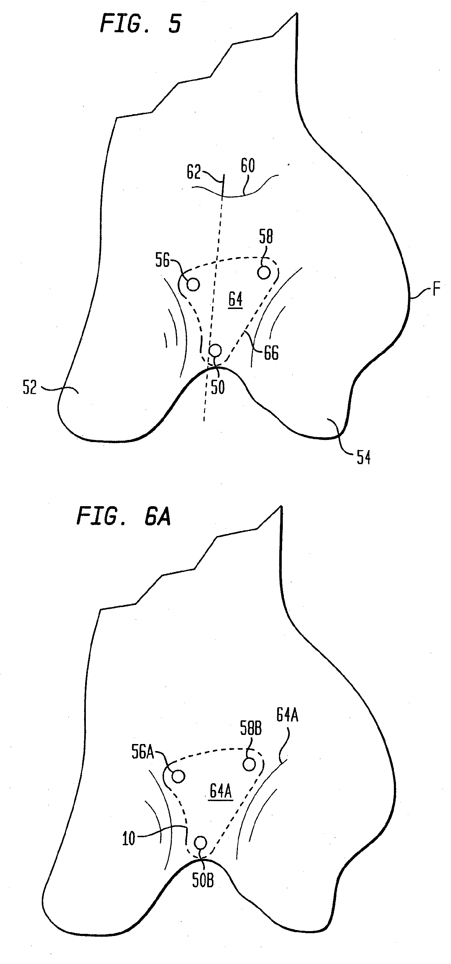

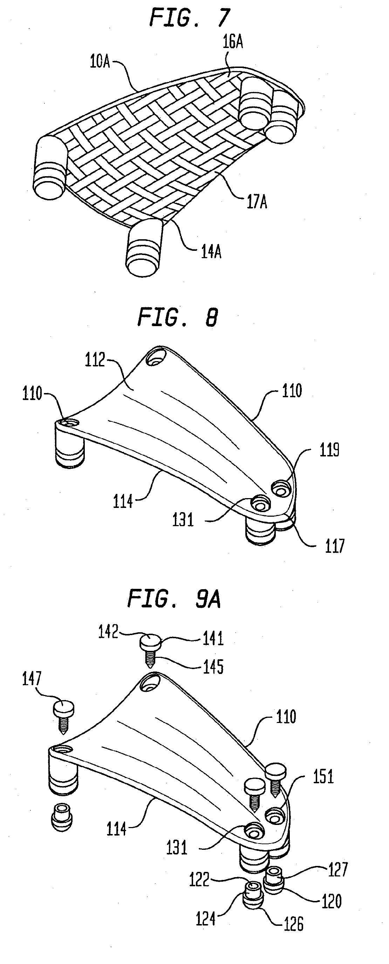

Once the implants 10 are constructed they may be implanted. In a method of operation, fixation of the flexible joint implant 10 to the bone is achieved by attaching pegs 20 to the underlying bone as shown in FIGS. 5 and 6. For instance, with regard to the trochlear groove, a hole 50 may be drilled in the femur F at a location proximate where the anterior cruciate ligament and the posterior cruciate ligament (not shown in the drawings) contacts the femur and between the lateral condyle 52 and medial condyle 54. Two additional holes 56 and 58 may be drilled proximate the anterior cortex edge 60 of the femur. Preferably holes 56 and 58 are separated by a distance such that a longitudinal axis 62 passing through the center of hole 50 parallel with a natural axis of the femur passes between the holes 56 and 58. Once the holes 50, 56 and 58 are drilled the shape of the flexible joint implant 10 may be carved out of the bone to form an implant-receiving area 64 denoted by the dashed lines. The depth of the implant-receiving area 64 is dependent on the implant, with an edge 66 of the receiving area 64 able to engage the edge 13 of the flexible joint implant 10. Of course, the implant-receiving area may carved out prior to the holes 50, 56 and 58 being formed.

With the femur prepared, the flexible joint implant 10 may be moved proximate the femur and specifically the trochlear groove, as shown in FIG. 6. The pegs 20 of the flexible joint implant 10 are aligned with the holes 50, 56 and 58 and received therein to thereby anchor the flexible joint implant to the femur. As the pegs 20, shown in hidden view, are aligned with the holes 50, 56 and 58 the pegs are pressed inwards until the bone-contacting surface 14 of the flexible joint implant 10 comes in contact with the femur. Specifically, the bone-contacting surface 14 contacts the implant-receiving area 64, which has been cut out and shaped to receive the flexible joint implant 10. The edge 13 of the flexible joint implant 10 is disposed adjacent an edge 66 of the implant-receiving area 64 when the implant 10 is placed correctly.

Preferably, the implant-receiving area 64 has a depth that enables the articulate-bearing surface 12 to be positioned at a height relative to the exterior surface of the femur F such that the articulate-bearing surface 12 approximates the positioning of a surface of a normal trochlear groove, enabling a patella (not shown in figures) to traverse along the flexible joint implant 10 as it would a normal femur.

In an alternate embodiment, as shown in FIG. 6A, the flexible joint implant 10 may just be blanketed onto an implant receiving area 64A. In such a situation, cartilage on the surface of a bone may or may not be removed from the implant receiving area 64A. As shown in FIG. 6A, a plurality of holes 50A, 56A and 58A may be drilled within the implant receiving area 64A. Once the implant receiving area 64A has been prepared, the flexible joint implant 10 may be positioned in the receiving area 64A with the anchoring mechanisms i.e., pegs 20 being received within holes 50A, 56A, and 58A. Thus, in this embodiment, the body 16 of the flexible implant 10 simply blankets a specific area as opposed to being embedded within a specific area as well as covering the area.

Referring to FIG. 7, a flexible joint implant 10A may be constructed similarly to flexible joint 10 but include a flexible porous metal underside 17A. The metal underside 17A is positioned against the bone-contacting surface 14A of body 16A. And the metal underside 17A may be constructed using methods discussed herein with respect to the anchoring mechanisms described herein. The metal underside 17A may include a polymer-engaging portion and a bone-engaging portion similar to pegs 20. The polymer-engaging portion of the metal underside 17A enables the body 16A to be engaged to the metal underside such that the two are affix to one another. And the bone-engaging portion permits and promotes bone ingrowth to thereby lock the metal underside 17A to the surrounding tissue. Thus, the metal underside 17A may function similar to pegs 20 described previously. In an alternate embodiment, the underside may be made from a polymer material and have a porous structure to promote soft tissue in-growth. Of course, a combination of the two may also exist.

In alternate embodiments not shown, the body 16A may be attached to the metal underside 17A using means that allow for the body 16A to be removeably attached to the metal underside. Such embodiments may include snap-fitting the two elements together, screwing them together and the like.

In one aspect of the present invention, the flexible joint implant is manufactured by attaching a flexible body to an anchoring mechanism in a temporary manner. For instance, as shown in FIGS. 8-9A, a flexible joint implant 110 may be similarly constructed as flexible joint implant 10 except that the body 116 is removably attached to pegs 120. The pegs 120 may include a bone contacting portion 126, an intermediate portion 124 and a protrusion 122 extending upwardly from the intermediate portion 124. The protrusion 122 may have a circular geometric shape, as shown in the figures, or any other exterior shape. The protrusion 122 includes an engaging element exposed at an internal surface of the protrusion such as internal threads 127. The internal threads 127 are adapted to mate with external threads of a screw as will be described below. The body 116 may be formed using a similar molding process as that discussed previously and the implant 110, as before includes an articulate-bearing surface 112 and a bone-contacting surface 114. The articulate-bearing surface 112 preferably has a shape that mimics the joint feature to which the flexible joint implant 110 is to be attached to, such as in this case, a trochlear groove. The flexible joint implant 110 may be formed using processes discussed herein, but rather than imbedding a part of the body 116 into a portion of the pegs 120, the body 116 includes a plurality of engagement elements 117, each having a through hole 119 extending from the articulate-bearing surface 112 through the bottom of the engagement elements 117. The through holes 119 may have internal threads 131 that are sized similar to the internal threads 127 of the protrusions 122 of pegs 120. The engagement elements 117 may be considered as part as the anchoring elements of the implant. The engagement elements 117 are preferably formed from the same material as the body 116.

In a method of implantation, as before, holes and an implant-receiving area are prepared within the joint, i.e., trochlear groove. Next, a plurality of pegs 120 is disposed within the holes positioned within the bone and pressed downward within the holes until reaching a desired position. The pegs 120 are positioned such that the protrusions 122 face out of the bone. The body 116 is then brought proximate the implant-receiving area. The engagement elements 117 of the body 116 are positioned within the holes drilled within the femur and translated into the holes until the engagement elements 117 are mated with the protrusion 122 of pegs 120. By pressing firmly down, the polymeric engagement elements 117 may be fitted snugly around the protrusions 127 in a male to female type relationship.

Next, in order to lock the body 116 to the pegs 120, a plurality of screws 141, each having a head 143 and a post 145 are received within the holes 119 of the body 116. The screws 141 preferably also have external threads 147 that may be engaged with the internal threads 131 of the holes 119 and the internal threads 127 of the protrusions 122. The screws 141 are tightened until the body 116 is anchored to the pegs 120 with the head 142 of the screws being received within recesses 151 of the body 116 such that the heads 143 of the screws do not create an abutment along the articulate-bearing surface 112 of the implant 110. The screws 141 are preferably sufficiently tightened so that the articulate-bearing surface 112 is planar with the rest of the surface of the trochlear groove and forms a surface equivalent to a normal trochlear groove. The articulate-bearing surface 112 is designed to slide against cartilage, bone and/or an additional implant.

By using a removable locking mechanism, if the flexible joint implant 110 should need to be replaced, rather than removing the entire implant, a surgeon would simply unscrew the screws 141 locking the body 116 to the pegs 120 and remove them from the structure while subsequently removing the body 116 of the flexible joint implant 110. Since the pegs 120 are still maintained within the holes of the implant-receiving area, a subsequent body may be coupled to the pegs 120, as previously described, thereby avoiding the need for a complete and entire new surgery.

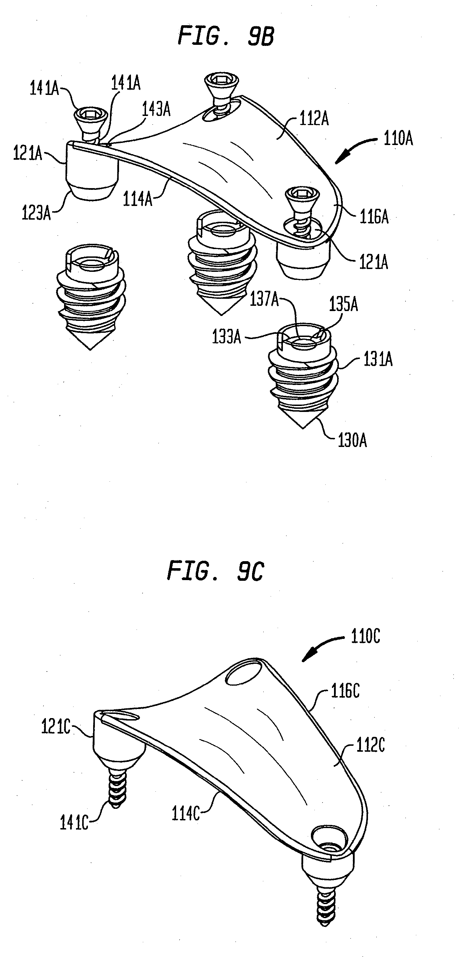

In an alternate embodiment, as shown in FIGS. 9B and 9C, the flexible joint implant may be constructed without a porous metal ingrowth feature. For instance, as shown in FIG. 9B, flexible joint implant 110A includes a body 116A having an articulate-bearing surface 112A and a bone-contacting surface 114A.

The joint implant 110A also may include a plurality of protrusions 121A extending outwardly from the bone-contacting surface 114A. Preferably, a hole 123A extends through each protrusion 121A all the way from the articulate-bearing surface 112A to a remote end 123A of the protrusions.

In a method of attaching the joint implant 110A to the femur and specifically adjacent to the trochlear groove, an implant-receiving area is prepared as discussed before. However, in order to attach the flexible joint implant 110A to the implant-receiving area, a plurality of cancellous bone screws 130A may be disposed within the implant-receiving area. The cancellous bone screws 130A include an external thread 131A and a hole 133A extending at least partially through the cancellous bone screws beginning at an open end 135A. The cancellous bone screws 130A preferably include internal threads 137A disposed within the internal holes 133A. The cancellous bone screws 130A are positioned within the implant-receiving area. They are embedded into the bone and provide an anchoring mechanism that enables the flexible joint implant 110A to be engaged and affixed to the bone. When the flexible joint implant 110A is disposed in the implant receiving area, the protrusions 121A of the flexible joint implant are aligned with the cancellous bone screws 130A. At least a portion of the protrusions 121A may be received within the openings 135A of the cancellous bone screws 130A.

Next, a plurality of screws 141A may be threaded through the protrusions 121A and into the openings 135A of the cancellous bone screws 130A. As the screws 141A are tightened, the external threads 143A of screws 141A engage the internal threads 137A of the cancellous bone screws 130A thereby locking the body 116A of the flexible joint implant 110A to the cancellous bone screws. Thus, the body 116A of the flexible joint implant 110A is removably attached to the cancellous bone screws 130A, which are embedded within the bone of the femur. This enables the body 116A to be removed from its engagement with the bone in order that it may be replaced if so required. This can be done without removing the anchors, i.e., the cancellous bone screws 130A.

As shown in FIG. 9C, flexible joint implant 110C is similarly constructed to flexible joint implant 110A and includes a body 116C having an articulate-bearing surface 112C and a bone-contacting surface 114C. As with the embodiment discussed in FIG. 9B, the flexible joint implant 110C also includes a plurality of protrusions 121C extending outwardly from the bone-contacting surface 114C. Each of the protrusions 121C include in a hole extending therethrough. In order to attach the flexible joint implant 110C an implant-receiving area is prepared in the femur. The implant-receiving area preferably includes recesses or holes for receiving the protrusions 121C. After the flexible joint implant 110C is mounted correctly within the implant-receiving area, a plurality of screws 141C may be disposed within the holes of the protrusions 121C and threaded into the bone. Thereby locking the flexible joint implant 110C to the femur or another bone.

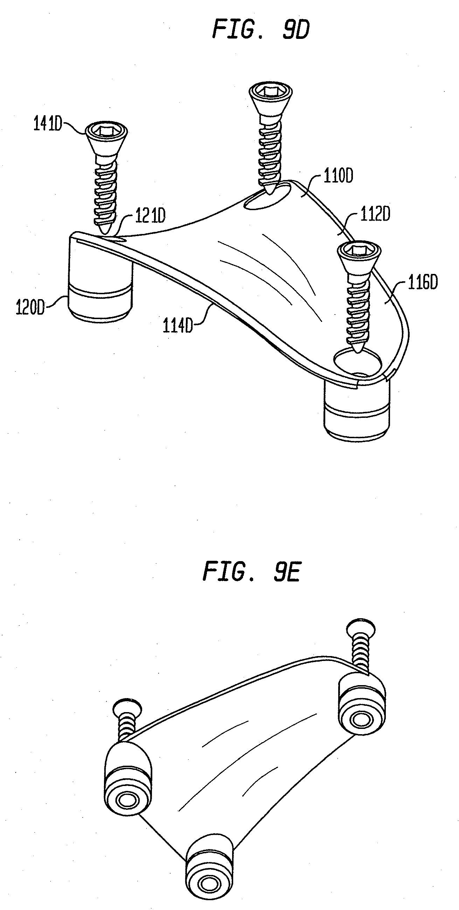

In yet another alternate embodiment as shown in FIGS. 9D and 9E, flexible joint implant 110D may be similarly constructed as flexible joint implant 110 and include a body portion 116D having an articulate-bearing surface 112D and a bone-contacting surface 114D as well as pegs 120D extending outwardly from the bone-contacting surface 114D. But in contrast to the flexible joint implant 10, flexible joint implant 110D also includes a plurality of holes extending through the pegs 120D, such that when the flexible joint implant 110D is received within the implant-receiving area a plurality of screws 141D may be inserted through holes 121D of pegs 120D. And the screws 141D may be driven into the bone so as to affix the flexible joint implant 110D immediately to the bone. This allows for an immediate fixation of the flexible joint implant 110D. Thereby providing a stable and relatively immovable object as bone ingrowth engages the pegs 120D.

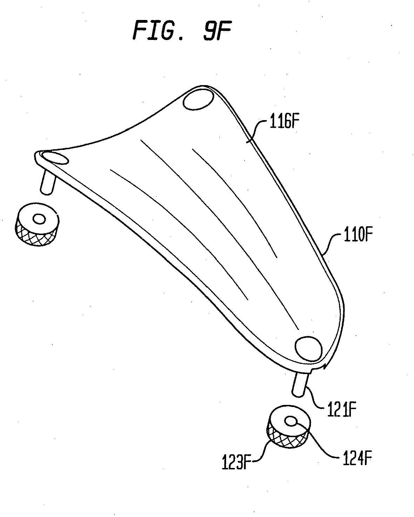

Further, as shown in FIG. 9F, the flexible joint implant 110F, may include a plurality of rods 121F that form part of the anchoring mechanisms. The rods 121F may be formed using methods similar to that used to form the pegs 20. And the body 116F of the implant 110F may be attached to the rods 21F similar to how they are attached to the pegs 20 described herein. In order to lock the implant 110F in place, a plurality of collars 123F may be positioned within bores within a bone. The collars 123F may be made from a metal and include a porosity that promotes bone ingrowth. Once the collars 123F as positioned within the bone, the rods 21F may be received within an aperture 124F of a particular collar 123F. The rods 121F may be snap-fitted into the collars or locked using various other engagement devices. In one embodiment, the inside of the collars include a polymer such that he collars may apply a tight engagement onto the rods 121F.

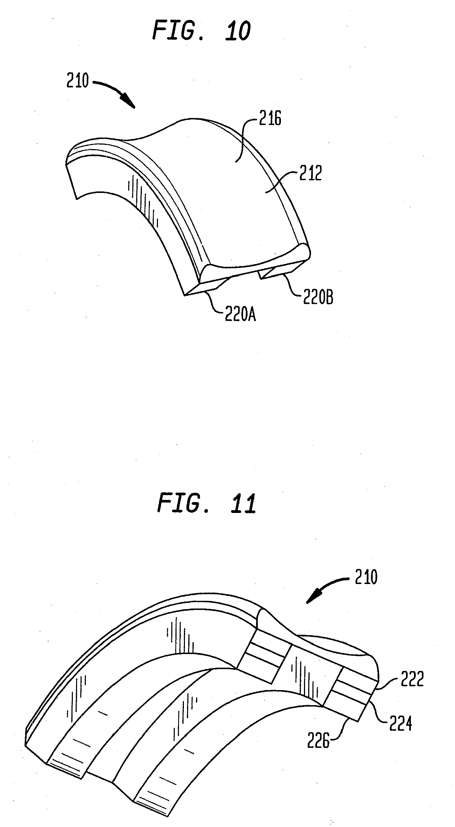

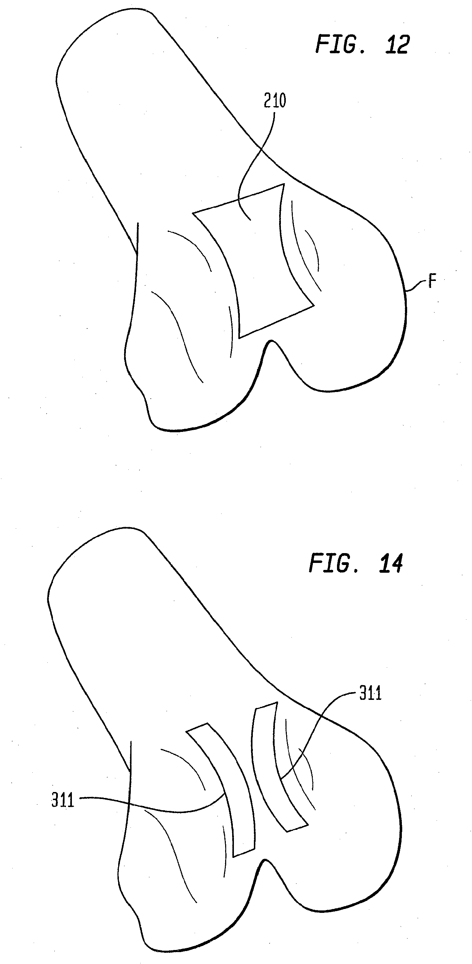

In one aspect of the present invention, rather than using of pegs as anchoring mechanisms, a flexible joint implant may include at least one rail such as that shown in FIGS. 10 and 11. The flexible joint implant 210 shown in FIGS. 10 and 11 is similar to flexible joint implant 10, except that rather than pegs, two rails 220A and 220B serve to anchor the flexible joint implant 210 to the femur F as shown in FIG. 12. The rails 220A and 220B may be similarly constructed as pegs 20 or 120 as previously discussed and preferably include a bone-contacting portion 226, an intermediate portion 224, and a polymer-engaging portion 222. Body 216 of flexible implant 210 is preferably comprised of a polymeric material such as polyurethane and may be created using a molding process to not only form the body 216, but also to embed the body 216 within the polymer-engaging portion 222 of respective rails 220A and 220B. As before, the flexible joint implant 210 should be entrenched within the femur deep enough such that the articulate-bearing surface 212 of the body 216 is positioned on the femur F so as to form a relatively smooth transition from the implant 210 to the bone such that the normal kinematics of the knee joint may be maintained. Specifically, the flexible joint implant 210 may be considered a trochlear groove implant, which enables the patella to traverse along the femur.

Although not shown in the figures, the flexible implant 210 may not only extend within the trochlear groove of the femur but also overlay at least one of the lateral or medial condyles.

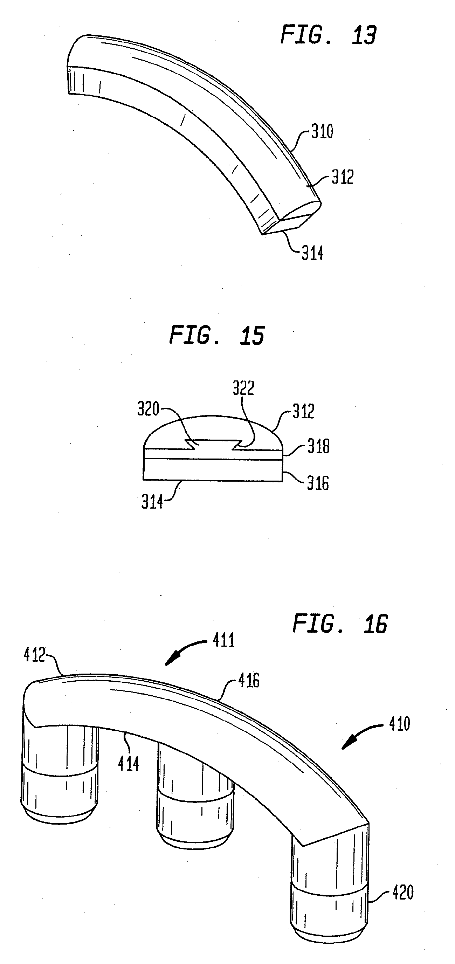

Still in another aspect of the present invention, a flexible joint implant 310, specifically adapted for reconstruction of the trochlear groove may be constructed as a single rail having an articulate-bearing surface 312 and a bone engaging portion 314 as shown in FIGS. 13-15. The bone engaging portion 314 may include a bone engaging surface 316 and a relatively solid intermediate portion 318. The bone engaging portion 314 may be constructed using methods discussed herein such as by selective laser sintering, selective laser melting, e-beams or other high energy sources and the like wherein the intermediate portion 318 consists of a relatively non-porous metal structure and the bone-engaging surface 316 consists of a metal structure that has a porosity that promotes bone ingrowth. The intermediate portion 318 may include a key 320 that can act as an anchoring mechanism for the articulate-bearing portion. With the bone-engaging portion constructed, the articulate-bearing portion 312 may be molded against the bone-engaging portion 314 similar to that discussed earlier. Once the articulate-bearing portion 312 is hardened, the key 320 locks the articulate-bearing portion 312 to the bone-engaging portion 314 as the key 320 fits within a groove 322 created in the articulate-bearing portion by the dimension of the key 320 itself. The key 320 or at least a top section of the intermediate portion 318 may be constructed having a porosity that allows the polymer of the articulate-bearing portion to imbed itself within the key 320 such that an anchoring mechanism is formed although this may not be required.

As shown in FIG. 14, two flexible joint implants 310, such as individual rails 311 may be placed along respective inner surfaces of the sides of the lateral and medial condyles of the femur so as to reconstruct the natural trochlear groove allowing the natural kinematics between the trochlear groove and the patella (not shown in the drawings). The flexible joint implants 310 are implanted into the femur as earlier discussed with a portion of the bone being scraped so as to form an implant-receiving area into which the flexible implants 310 are placed. The flexible implants 310 are preferably placed deep enough within the femur such that the articulate-bearing portion 312 forms a smooth and continuous surface with the parts of the femur that are not scraped away such that the normal kinematics of the knee joint may be achieved. Although the rails 311 are shown positioned along the sides of the trochlear groove adjacent the medial and lateral condyles, a rail 311 may also be placed within the sulcus of the trochlear groove. Various combinations of the placement of the rails may include only positioning a single rail 311 or multiple rails. Further, although the rails 311 are shown extending in a direction from the anterior cortex to the intercondular notch, the rails 311 may also extend in a direction from a lateral condyle to a medial condyle.

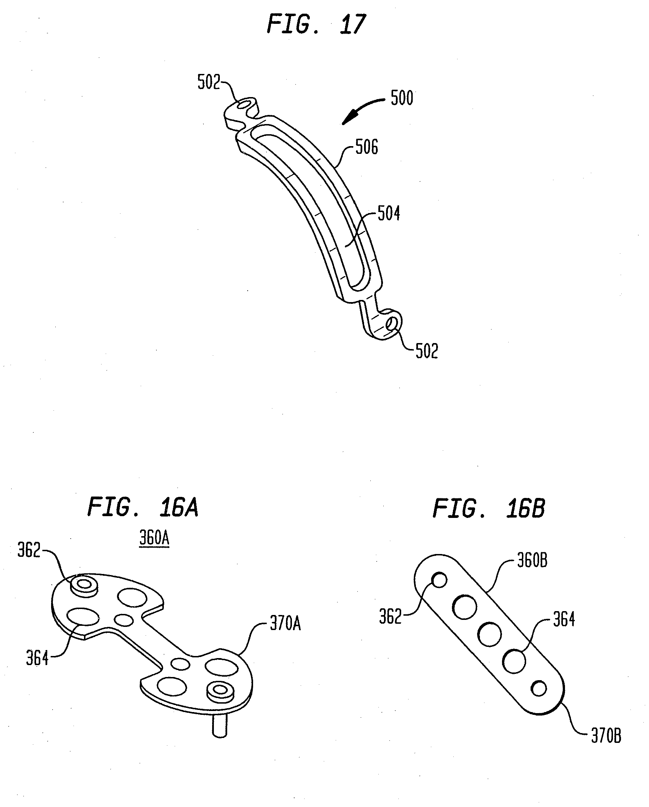

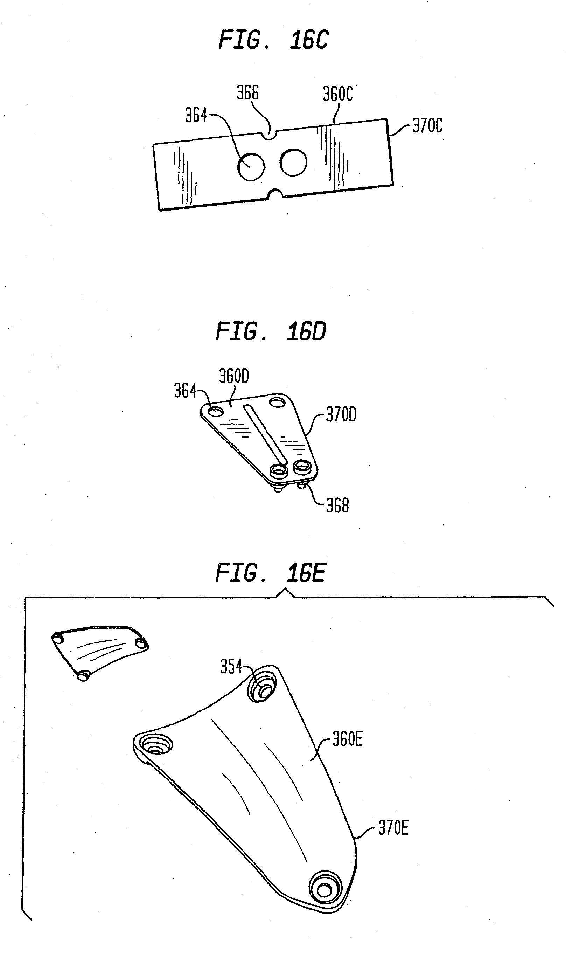

In one aspect of the present invention, an individual rail may be constructed as shown in FIG. 16. FIG. 16 illustrates flexible joint implant 410 comprised of an individual rail 411. The individual rail 411 includes an articulate-bearing surface 412 and a bone-contacting surface 414 comprising a body 416. The rail 411 also includes a plurality of pegs 420 extending outwardly from the bone-contacting surface 414. The pegs 420 may be similarly constructed as pegs 20 or various other anchoring mechanisms described herein, including embodiments discussed with regard to using screws to attach the implant to a bone. In order to implant individual rail 411, an implant-receiving area may be prepared as discussed with flexible joint implant 310. The implant-receiving area preferably has a plurality of recesses or depressions that are adapted to receive pegs 420 therein while allowing bone-contacting surface 414 to be disposed adjacent to the femur. Preferably, when the flexible joint implant 410 is affixed to the bone the articulate-bearing surface 412 forms a continuous surface with the femur and specifically with the trochlear groove.

As we have discussed various flexible joint implants that may be implanted into various bones including specifically the femur and more specifically the trochlear groove of the femur, various instruments may be provided for conducting the surgery. One such aspect includes providing a flexible drilled template design such as that shown in FIGS. The templates 360A, 360B, 360C, 360D and 360E may include fixation holes 362, drill holes 364, relief cuts 366 as well as positioning features 368. The fixation holes 362 enable the individual drill template to be temporarily anchored to the femur such that holes in the femur may be drilled using the drill holes 364 as a template so that the correct required alignment for the holes in the femur may be achieved. The correct alignment for the holes in the femur is required otherwise the particular flexible joint implant may not perfectly fit the implant-receiving area that has been carved out of the bone. The outer edges 370A - 370E of the individual drill templates 360A to 360E may be traced so as to form a pattern on the particular bone, i.e., the femur, which can then be carved out of the bone such that the flexible joint implant will fit tightly within the respective implant-receiving area. The locating pegs 368 enable the flexible drill template to be temporarily anchored within holes already constructed in the femur or bone. Thereby allowing the rest of the template to be traced and pre-drill holes to be formed so that a correct alignment may be achieved.

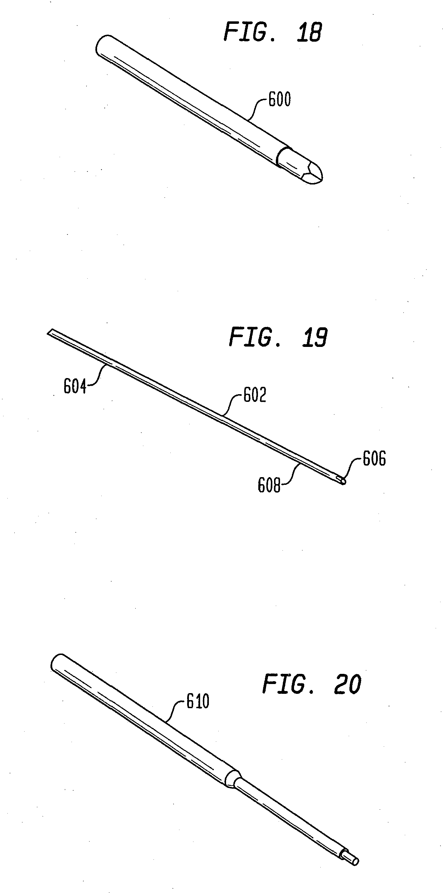

As shown in FIG. 17 a burr template 500 may be provided. The burr template 500 may be anchored to the femur using screws, pins or other holding devices (not shown) extending through holes 502 of the burr template. The burr template 500 is preferably anchored at its correct position and includes an anterior recess 504 for forming the implant receiving an area. The recess 504 may be defined by various walls 506 of the burr template 500. In the embodiment shown in FIG. 17, the burr template is designed for preparing an implant-receiving area for an individual rail such as that shown in FIGS. 13 and 16. However, various burr templates may also be provided for the specific shape of the implant being used. Once the burr template 500 is attached to the bone a cutting device such as that shown in FIG. 18 may be used to chisel away the bone that is exposed in recess 504 to form an implant-receiving area. As stated before, the flexible bone implant preferably is sized to fit within the recess 504 such that once the femur or other bone is prepared, the flexible implant may fit within the cut out portion.

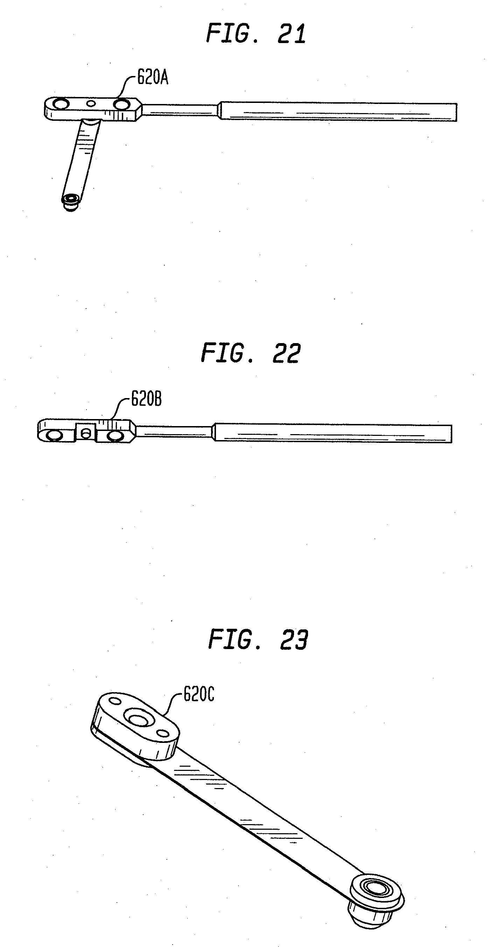

FIGS. 18 - 21 illustrate some of the tools that may be used in order for implanting the flexible joint implants discussed herein. For instance, FIG. 18 illustrates a cutting instrument 600 that may be used to remove small pockets of bone from the implant-receiving area for implant fixation. In addition, once the local areas of the bone are removed, a flexible joint implant may be inserted through a small incision with the aid of sutures and a needle shown in FIG. 14. The needle 602 includes a long extension 604 and a hole 606 disposed at a first end 608 of the needle. Similarly, the bone fixation pegs may be inserted using an impacting device 610, such as that shown in FIG. 20. FIGS. 21-23 illustrate alternate embodiments of the drill templates which include a handle. The templates 620A-620C may be attached to an external tool such that it is held in place against the femur once correctly positioned. Various holes and recesses may be constructed in a bone using the template 620A-620C as guides.

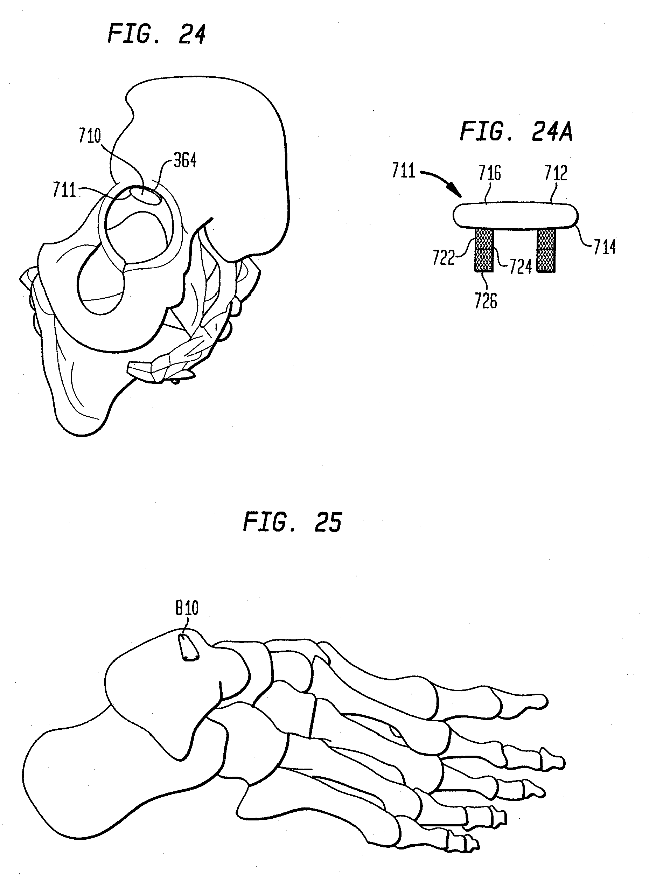

Some of the different joints where the present invention is also suitable include the acetabulum, an ankle joint, a patella, femoral head and a humeral head as shown in FIGS. 24-28.

For instance, in FIG. 24, the flexible joint implant 710 is in the shape of a circular plug 711. The flexible joint implant 710 may also have other geometric shapes as desired. The circular plug 711 may be placed within an implant-receiving area 764 of the acetabulum that is created by removing portions of the bone. The circular plug 711 may have an articulate-bearing surface 712 and a bone-contacting surface 714 comprising a body 716 made from a polymer material.

As with additional embodiments discussed herein, the circular plug 711 has a plurality of anchoring mechanisms such as pegs 720, that include a polymer-engaging portion 722, an intermediate portion 724 and a bone-ingrowth portion 726 as described herein. In the embodiment shown in FIGS. 24 and 24A, the polymer-engaging portion 722 has a gradient profile. A gradient profile refers to the porosity of the structure and specifically the gradient profile in peg 720 includes a porosity that increases as you become more removed from the intermediate portion 724. The bone-engaging portion 726 also has a gradient profile such that the porosity of the preferably metal structure has a porosity that increases as you are more removed from the intermediate portion 724. Of course the gradient profile may be reversed for both structures or may even be random, thereby vary from location to location.

As shown in FIG. 25 the flexible joint implant 810 may be used near the ankle joint. Flexible joint implant 810 may be similar constructed to embodiments discussed herein.

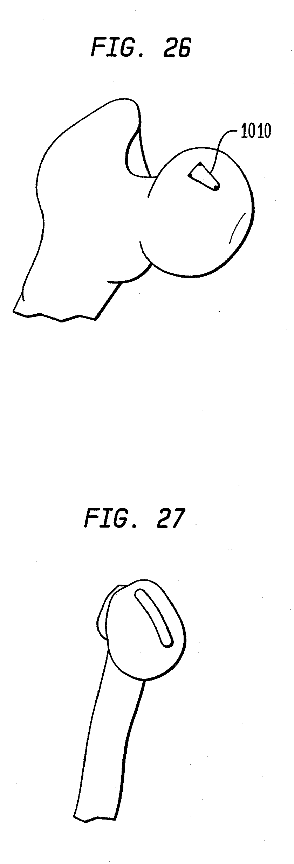

Further, a flexible joint implant 1010 may be used to form a portion of a femoral head stem, as shown in FIG. 26 or humeral head as shown in FIG. 27.

In alternate embodiments, the anchoring mechanisms or pegs may be at least temporarily attached to the bone using a polymer. For instance, an inner core of a peg may be filled with a resorbable polymer during implantation. The polymer may then be melted such the polymer melt leeches out of the peg and into various crevices within the surrounding bone. As the polymer cools in interlocks the peg to the bone. Over time, the polymer absorbs into the body of the patient and is replaced by bone ingrowth.

In yet another alternate embodiment, to temporary lock a peg to the surrounding bone, a polymer may be introduced into a void of the peg. Once introduced, the polymer may be heated such as by UV light, thereby causing the polymer to melt and engage the surrounding bone. An adhesive may replace the polymer or be dispersed within the polymer for the process as well.

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A trochlear groove implant comprising:
a body having an articulate-bearing surface, a bone-facing surface and an edge extending therebetween, said articulate-bearing surface configured to mimic a portion of a normal trochlear groove;
at least one anchoring mechanism extending outwardly from said body, said at least one anchoring mechanism being at least partially attached to said body, wherein said at least one anchoring mechanism may be disposed within a bone to anchor said body to the bone.

2. The trochlear groove implant according to claim 1, wherein said articulate-bearing surface has a shape similar to a substantial portion of the trochlear groove, such that when the trochlear groove implant is implanted it extends from a lateral side of the trochlear groove to a medial side of the trochlear groove of a patient.

3. The trochlear groove implant according to claim 1, wherein said body of the implant substantially extends from an anterior cortex of the femur to an intercondular notch of the femur.

4. The trochlear groove implant according to claim 1, wherein said body of the implant is positioned within a sulcus of a trochlear groove.

5. The trochlear groove implant according to claim 1, where said body is shaped to similar to a portion of an articulating surface of a lateral side of a trochlear groove or a medial side of the trochlear groove such that said body of the implant may be implanted adjacent to either a lateral side of a trochlear groove or a medial side of a trochlear groove and said articulate-bearing surface of said body extends along only one side of the trochlear groove.

6. The trochlear groove implant according to claim 1, wherein said at least one anchoring mechanism includes a first portion for engaging said body and a second portion that has a porosity sufficient to permit bone ingrowth.

7. The trochlear groove implant according to claim 1, wherein said at least one anchoring mechanism includes a first portion integrally formed to said body and a second portion removeably attached to said first portion, wherein said second portion may be disposed within a recess created in a trochlear groove and said first portion is removably engaged to said second portion such that if so required said first portion may be disengaged from said second portion and said body including said first portion can be removed.

8. The trochlear groove implant according to claim 7, wherein said second portion includes a screw, wherein said body includes a hole extending from said articulate-bearing surface to said bone-facing surface, said hole further extending through said first portion, said first portion having threads disposed therein, wherein said screw engages said threads and is translated downward so as to affix said first portion to a bone.

9. The trochlear groove implant according to claim 1, wherein said body includes at least one hole extending from said first surface to said second surface, said at least one hole further extending through a peg.

10. The trochlear groove implant according to claim 1, wherein said at least one anchoring mechanism includes a key and said body of said implant includes a key-way to be engaged with said key of said anchoring mechanism.
